(19) Europäisches Patentamt

European Patent Office

Office européen des brevets

(11) Veröffentlichungsnummer: **0 201 004 B1**

(12) # EUROPÄISCHE PATENTSCHRIFT

(45) Veröffentlichungstag der Patentschrift: **24.03.93**

(51) Int. Cl.⁵: **A61K 39/40**

(21) Anmeldenummer: **86105706.5**

(22) Anmeldetag: **25.04.86**

(54) **Polyvalentes Hyperimmunglobulin-Präparat.**

(30) Priorität: **04.05.85 DE 3516119**

(43) Veröffentlichungstag der Anmeldung:
**17.12.86 Patentblatt 86/46**

(45) Bekanntmachung des Hinweises auf die Patenterteilung:
**24.03.93 Patentblatt 93/12**

(84) Benannte Vertragsstaaten:
**AT BE CH DE FR GB IT LI LU NL SE**

(56) Entgegenhaltungen:
**EP-A- 0 129 147**
**FR-A- 2 160 542**

**CHEMICAL ABSTRACTS, Band 102, Nr. 3, 21. Januar 1985, Seite 521, Zusammenfassung Nr. 22552h, Columbus, Ohio, US; M.J. NELLES et al.: "Mouse monoclonal antibodies reactive with J5 lipopolysaccharide exhibit extensive serological cross-reactivity with a variety of Gram-negative bacteria", & INFECT. IMMUN. 1984, 46(3), 677-81**

**BIOLOGICAL ABSTRACTS, Band 76, 1983, Zusammenfassung Nr. 49375; E.J. ZIEGLER et al.: "Treatment of gram-negative bacteremia and shock with human antiserum to mutant Escherichaia coli", & N. ENGL. J. MED. 307(20), 1225-1230, 1982**

(73) Patentinhaber: **Biotest Pharma GmbH**
**Flughafenstrasse 4**
**W-6000 Frankfurt 71(DE)**

(72) Erfinder: **Stephen, Wolfgang, Dr.**
**P. Holzmannstrasse 84**
**W-6072 Dreieich(DE)**
Erfinder: **Dichtelmüller, Herbert, Dr.**
**Rossertstrasse 14**
**W-6231 Sulzbach/Ts.(DE)**
Erfinder: **Kloft, Michael, Dr.**
**Kollwitzweg 27**
**W-6100 Darmstadt(DE)**

(74) Vertreter: **Dr. Fuchs, Dr. Luderschmidt Dr. Mehler, Dipl.-Ing Weiss Patentanwälte**
**Abraham-Lincoln-Strasse 7**
**W-6200 Wiesbaden (DE)**

**Beschreibung**

Die Erfindung betrifft ein neues polyvalentes Hyperimmunglobulin-Präparat mit Aktivitäten gegen gram-negative und gram-positive Keime und ein Verfahren zur Gewinnung dieses polyvalenten Hyperimmunglobulin-Präparats.

Die serologischen Unterschiede zwischen Bakterien werden durch oberflächengebundene, spezies-spezifische Antigene bestimmt. Daraus resultiert, daß spezifische Antikörper, zum Beispiel gegen Salmonella, nicht oder nur sehr schwach an Antigene anderer Keime, z. B. Pseudomonas binden. Die Folge hieraus ist, daß zur Therapie und Prophylaxe von bakteriellen Infektionen für jeden speziellen Keim ein spezielles Hyperimmunglobulin bzw. Hyperimmunserum benötigt wird. Dies schränkt die Anwendung von Hyperimmunglobulinen wesentlich ein.

Andererseits ist bekannt, daß die Gruppe der gram-negativen Keime (Salmonella, E. coli, Serratia, Pseudomonas u. a. ein gemeinsames Antigen besitzen. Von Braude und Ziegler*wurde gegen dieses Antigen gerichtetes Immunserum gewonnen und erfolgreich bei Patienten mit gram-negativen Infektionen eingesetzt. Dieses Hyperimmunserum wurde durch Immunisierung von freiwilligen Spendern mit einem Impfstoff der E. coli Mutante J5 gewonnen. Es ist also bekannt, daß man durch Vakzinierung von Spendern mit einem Impfstoff der E. coli Mutante J5 ein gegen das den gram-negativen Keimen gemeinsame Antigen wirksames Immunserum gewinnen kann.

Aus der EP-A-0129 147 ist ferner bekannt, daß aus dem normalen Plasma von Spendern ein gegen Pseudomonas gerichtetes Hyperimmun-Globulin-Präparat auch dann gewonnen werden kann, wenn diese Spender zuvor nicht gegen Pseudomonas vakziniert worden waren oder sich in jüngerer Zeit eine Pseudomonas-Infektion zugezogen hatten. Das dafür verwendete Plasma mußte nur nach überdurchschnittlich hohen Antikörpertitern gegen Pseudomonas gescreent werden. Es zeigte sich, daß Serum mit überdurchschnittlich hohem Antikörpertiter gegen Antigene von 4 Fisher-Immunotypen von Pseudomonas Aeruginosa genügte, um ein Hyperimmun-Präparat zu gewinnen, welches Aktivität gegen alle 7 Fisher-Immunotypen aufwies.

Ein Ziel der vorliegenden Erfindung ist daher die Gewinnung einer Immunglobulin-Präparation mit Aktivitäten gegen gram-negative Keime, das gewonnen werden kann, ohne daß freiwillige Spender zuvor mit E. coli J5 immunisiert werden müssen.

Ein weiteres Ziel dieser Erfindung ist eine Immunglobulinpräparation, die auch Aktivitäten gegen gram-positive Keime aufweist.

Diese Ziele werden mit der vorliegenden Erfindung erreicht.

Bestimmt man die Antikörper gegen das E. coli J5-Antigen in nicht immunisierten Plasmapherese-Spendern, so findet man, daß auch normale Populationen nicht vakzinierter Personen Aktivitäten gegen das der Gruppe der gram-negativen Keime gemeinsame Antigen besitzen, d. h. daß Antikörper gegen das E. coli J5-Antigen natürlich vorkommen, so daß ein Spenderstamm mit hohen Titern gegen E. coli J5-Antigen ermittelt werden kann. Bestimmt man jedoch das ganze Antikörperspektrum solcher Plasmapheresespender mit hohen Aktivitäten gegen E. coli J5, so zeigt sich überraschenderweise, daß auch die Antikörperaktivitäten gegen andere gram-negative Keime deutlich erhöht sind. In einer Spenderpopulation mit niedrigen Titern gegen E. coli J5 ist dies nicht der Fall; hier (Abb. 1) zeigt lediglich ein Spender unter 10 erhöhte Titer gegen andere Keime.

Neben den Titern gegen gram-negative Keime sind jedoch überraschend die Titer gegen gram-positive Keime ebenfalls deutlich erhöht. Dies ist umso erstaunlicher, als gram-positive Keime sich von gram-negativen Keimem durch eine grundsätzlich andere Wandstruktur unterscheiden.

Während hohe Titer gegen E. coli J5 eines Plasmas mit hohen Titern gegen andere Keime korrelieren, kann andererseits ein hoher Titer (1 :320) gegen Pseudomonas mit niedrigem Titer gegen E. coli J5 (1:20) oder gegen Staphylococcus ( < 10) verbunden sein. Ebenso wurde gefunden, daß Plasma einen Titer von 1:160 gegen E. coli und gleichzeitig von < 10 gegen Staphylococcen und Streptococcen aufweisen kann. Daraus ergibt sich, daß vor allem ein Screening von Plasma auf hohe Titer gegen E. coli J5, nicht aber gegen andere Antigene zu einer Auswahl von Plasmen führt, die sowohl gegen gram-negative als auch gram-positive Keime hohe Antikörpertiter aufweisen.

Mittels eines Screenings unter Plasmapheresespendern mit erhöhtem Titer gegen E. coli J5 lassen sich somit auch solche Spender mit erhöhten Titern gegen andere gram-negative Keime und auch gegen gram-positive Keime ermitteln.

Neben dem E.coli J5-Antigen gibt es in der Gruppe der gram-negativen Bakterien ein weiteres gemeinsames Antigen, Lipid A, welches ebenfalls Teil der Antigenstruktur dieser Bakterien ist. Lipid A ist

* Ziegler et al., The New England Journal of Medicine 307 , 1982, S. 1225 bis 1230

zudem für toxische Reaktionen (Pyrogenität) verantwortlich. Mittels eines Screenings unter Plasmapheresespendern mit erhöhtem Titer gegen Lipid A lassen sich somit auf die gleiche Weise, wie es vorstehend im Zusammenhang mit dem E.coli J5-Antigen beschrieben wurde, Spender mit erhöhten Titern gegen andere gram-negative und gram-positive Keime ermitteln.

Stellt man aus dem Plasma solcher Spender, z. B. über die Cohn-Fraktion II/III bzw. III nach bekannten Verfahren eine Immunglobulin-Präparation her, ergibt sich ein Präparat, welches sich durch hohe Titer gegen gram-negative und -positive Keime auszeichnet. Tabelle 1 zeigt diese Titer im Vergleich mit einem herkömmlichen Immunglobulin-Präparat.

Untersucht man die derart gewonnene Immunglobulin-Präparation, so findet man, daß sie neben IgG als Hauptbestandteil noch wesentliche Mengen an Antikörpern der Klassen IgA und IgM enthält. IgG und IgA sind im allgemeinen 7s-Moleküle; IgM ist im allgemeinen ein 19s-Molekül (s. Lexikon Biochemie, Leipzig 1976, S. 509). Auch die in bekannter Weise erhaltenen Bruchstücke der vorstehend genannten Moleküle können in erfindungsgemäßen Präparationen enthalten sein.

Auf diese Weise wird ein Immunglobulin-Präparat gewonnen, welches gegen ein breites Spektrum von bakteriellen Infektionen, auch Mischinfektionen, eingesetzt werden kann und gleichzeitig Aktivitäten besitzt, die speziellen Hyperimmunglobulinen gegen unterschiedliche Keime entsprechen, und das somit ein polyvalentes Hyperimmunglobulin-Präparat darstellt.

Die Erfindung wird anhand der nachstehenden Ausführungsbeispiele näher erläutert.

Beispiel 1:

Plasmen wurden mittels das Passiven Hämagglutinationstestes auf hohe Titer gegen E. coli J5 gescreent. Dazu wurde als Antigen der Überstand einer autoklavierten Übernachtkultur von E. coli J5 (ATCC 39041) verwendet.

Plasmen mit reziproken Titern ab 1:320 wurden ausgewählt und vereinigt.

4240 ml dieses Plasmapools wurden nach Cohn fraktioniert, wobei die Fraktion II/III mittels Octansäure und Calziumphosphat aufgearbeitet wurde. Erhalten wurden 207 ml einer 5 %igen Immunglobulinlösung, die mittels $\beta$-Propiolacton für die intravenöse Applikation modifiziert wurde. Nach intensiver Diafiltration und anschließender Sterilfiltration wurden ca. 180 ml Endprodukt erhalten.

Das Präparat enthält 4160 mg/100 ml IgG, gemessen mittels Kallestad Quantiplate, 480 mg/100 ml IgA und 960 mg/100 ml IgM. Die Bestimmung der Antikörpertiter in der passiven Hämagglutination ergab die in Tabelle 1 wiedergegebenen Werte.

Beispiel 2:

Entsprechend der Aufarbeitung von Beispiel 1 wurden aus 4290 ml gescreentem Plasma 200 ml IgM-angereicherte Immunglobulinlösung für die intravenöse Applikation gewonnen.

Beispiel 3:

Aus 4,35 Litern gesreentem Plasma wurden entsprechend Beispiel 1, jedoch über die Cohnfraktion III, 50 ml einer IgM-angereicherten Immunglobulinlösung (5%) gewonnen. Die Antikörper gegen Lipid A waren in dieser Präparation etwa 20- bis 38-fach höher als in einem Serumpool von ungesreenten Blutspendern.

Beispiel 4:

Aus 2,4 Litern gescreentem Pasma wurden 1,4 Liter einer stabilisierten Serumkonserve durch Behand-lung mit $\beta$-Propiolacton, UV und Aerosil$^R$ gemäß dem in der EU-A-14 333 beschriebenen Verfahren hergestellt. Auch hier waren die Antikörpertiter gegen Lipid A etwa 20- bis 40-fach höher als in einem Plasma von ungesreenten Blutspendern. Die Antikörpertiter dieses Präparats gegen verschiedene andere Antigene sind in Tabelle 2 wiedergegeben.

Tierexperimentelle Erprobung

Zur Bestimmung der Wirksamkeit des aus gescreentem Plasma hergestellten polyvalenten Hyperimmunglobulins wurden 0,5 ml einer 5 %igen Lösung Mäusen intravenös verabreicht, welche nun mit $10^7$ KBE(koloniebildenden Einheiten)von Pseudomonas aeruginosa pro Tier intraperitoneal infiziert wurden. Als Vergleich diente eine Gruppe von Tieren, welche Immunglobulin aus ungescreentem Plasma erhielten. In der Kontrollgruppe blieben die infizierten Tiere unbehandelt. Jede Gruppe umfaßte 21 Tiere. 25 Stunden nach Infektion überlebten in der unbehandelten Kontrollgruppe nur 14,3 % der Tiere. Durch Immunglobulin aus ungescreentem Plasma wurden 47,6 % der Tiere (= 10 von 21) geschützt; in der Gruppe der mit Immunglobulin aus gescreentem Plasma behandelten Tieren wurden 71,4 % (= 15 von 21 Tieren) geschützt (Tabelle 3). Somit sind in der mit Immunglobulin aus ungescreentem Plasma behandelten Gruppe nahezu doppelt so viele Tiere infektionsbedingt verendet wie in der mit Immunglobulin aus gescreentem Plasma behandelten Gruppe. Das Präparat weist somit eine erheblich bessere Wirksamkeit auf.

Tabelle 1    Reziproke Antikörpertiter (PHA gegen gram-
negative und gram-positive Keime.
Vergleich von drei Immunglobulinlösungen.

| Antigen | Reziproke Titer PHA | | |
|---|---|---|---|
| | Präparat 1 | Präparat 2 | Präparat 3 |
| E. coli J5 | 320 (40) | 1280 | 2560 |
| E. coli | 1280 | 1280 | 5120 |
| Ps. aerug. | 1280 | 10240 | 5120 |
| Klebs.pneu. | 640 | 2560 | 640 |
| Staph. aur. | 80 | 640 | 320 |
| Enterococc. | 80 | 640 | 320 |
| Str. pyog. | 160 | 320 | 160 |
| Str.virid. | 160 | 160 | 320 |
| Pneumoc. | n.d. | n.d. | 80 |

Präparat 1: IgM-angereicherte Immunglobulinlösung aus
nicht gescreentem Plasma

Präparate 2 Aus E. coli J5-gescreentem Plasma hergestellte,
und 3:    IgM-angereicherte Immunglobulinlösung.

Tabelle 2:Reziproke Titer gegen bakerielle Antigene in Serumkonserve

| Präparat | Antigen | | | |
|---|---|---|---|---|
| | E.coli | Ps.aerug. | Klebs.pneum. | Staph.aureus |
| Serumkonserve aus gescreentem Plasma | 320 | 1280 | 320 | 160 |
| Serumkonserve aus ungescreentem Plasma | 80 | 80 | 40 | 40 |

Tabelle 3    Wirksamkeit von Immunglobulinlösungen gegen
             Pseudomonas aeruginosa-Infektion

             Überleben von Pseudomonas aeruginosa-infizier-
             ten Mäusen 24 h nach Infektion

|  |  | Überleben |  | Verendete Tiere |
|---|---|---|---|---|
| Behandlung | $N_{ü}/N_O$ | | % | $N_v/N_O$ |
| unbehandelte Kontrolle | 3/21 | | 14,3 | 18/21 |
| Immunglobulin aus ungescreentem Plasma | 10/21 | | 47,6 | 11/21 |
| Immunglobulin aus E.coli J5 gescreentem Plasma | 15/21 | | 71,6 | 6/21 |

$N_{ü}$ = Anzahl überlebender Tiere

$N_v$ = Anzahl verendeter Tiere

$N_O$ = Gesamtzahl zu Versuchsbeginn

Abb. 1  Korrelation zwischen Titer gegen E. coli J5 und Titer gegen gram-negative und gram-positive Keime

**Patentansprüche**

1.  Polyvalentes Hyperimmunglobulin-Präparat mit Aktivitäten gegen gram-negative und gram-positive Keime, hergestellt nach herkömmlichen Methoden der Immunglobulin-Präparation aus einem Plasma- und/oder Serum- und/oder Vollblutpool, welcher aus dem Blut zuvor nicht vakzinierter Plasmapherese-Spender mit überdurchschnittlich hohem Titer gegen das E. coli J5-Antigen oder gegen das Lipid A-Antigen erhalten wurden.

2.  Polyvalentes Hyperimmunglobulin-Präparat nach Anspruch 1, dadurch gekennzeichnet, daß es neben IgG als Hauptbestandteil noch Antikörper der Klassen IgA und IgM enthält.

3. Polyvalentes Hyperimmunglobulin-Präparat nach Anspruch 2, dadurch gekennzeichnet, daß es IgG, IgM und IgA als 7s- und 19s-Moleküle enthält oder als auf bekannten Wegen erhaltene Bruchstücke derselben.

4. Verfahren zur Herstellung eines polyvalenten Hyperimmunglobulin-Präparats mit Aktivitäten gegen gram-negative und gram-positive Keime nach Anspruch 1, dadurch gekennzeichnet, daß das Plasma und/oder Serum und/oder Vollblut zuvor nicht vakzinierter Spender nach seinem Titer gegen das E. coli J5-Antigen oder gegen das Lipid A-Antigen gescreent wird, das Plasma und/oder Serum und/oder Vollblut mit überdurchschnittlich hohem Titer gegen das E. coli J5-Antigen oder gegen das Lipid A-Antigen vereinigt wird und aus dem so erhaltenen Pool nach herkömmlichen Methoden der Immunglobulin-Präparation das polyvalente Hyperimmunglobulin-Präparat gewonnen wird.

5. Verfahren nach Anspruch 4, dadurch gekennzeichnet, daß als Ausgangsmaterial Plasma und/oder Serum und/oder Vollblut verwendet wird, deren Titer einem Wert von mindestens 1:160 in der passiven Hämaglutination entspricht.

**Claims**

1. Polyvalent hyperimmunoglobulin preparation having activities against gram-negative as well as gram-positive agents, being prepared according to conventional immunoglobulin preparation methods from a pool of plasma and/or serum and/or whole blood, the said pool being obtained from the blood of previously non-vaccinated donors for plasmapheresis exhibiting a titer higher than average against the J-5 antigen of E.coli or against lipid A-antigen.

2. Polyvalent hyperimmunoglobulin preparation according to claim 1, characterized in that it contains antibodies of the Classes IgA and IgM in addition to IgG antibodies as main constituent.

3. Polyvalent hyperimmunoglobulin preparation according to claim 2, characterized in that it contains IgG, IgM and IgA as 7s- and 19s-molecules or as fragments thereof obtained according to known methods.

4. Method to prepare a polyvalent hyperimmunoglobulin preparation having activities against gram-negative as well as gram-positive agents according to claim 1, characterized in that the plasma and/or serum and/or whole blood of previously non-vaccinated donors is screened according to the titer against the J5-antigen of E.coli or against the lipid A-antigen, the plasma and/or serum and/or whole blood exhibiting a titer higher than average against J5-antigen of E.coli or agianst the lipid A-antigen is combined and in that the hyperimmunoglobulin preparation is prepared from a thus obtained pool in accordance with conventional methods for preparing immunoglobulin preparations.

5. Method according to claim 4, characterized in that plasma and/or serum and/or whole blood is used as starting material, the titer thereof exhibiting at least a value of 1:160 of passive hemagglutination.

**Revendications**

1. Préparation polyvalente d'hyperimmunoglobulines présentant une activité vis-à-vis des germes à Gram négatif et à Gram positif, caractérisée en ce qu'elle est produite selon les procédés traditionnels de préparation des immunoglobulines, à partir d'un pool de plasma et/ou de sérum et/ou de sang entier, qui a été obtenu à partir du sang de donneurs en plasmaphérèse, non vaccinés préalablement, avec des titres dépassant la moyenne d'anticorps anti-antigène J5 d'E. coli ou anti-antigène lipide A.

2. Préparation d'hyperimmunoglobulines polyvalente selon la revendication 1, caractérisée en ce que, outre l'IgG qu'elle contient à titre de composant principal elle contient également des anticorps appartenant aux groupes des IgA et IgM.

3. Préparation d'hyperimmunoglobulines polyvalente selon la revendication 2, caractérisée en ce qu'elle contient des IgG, IgM et IgA sous forme de molécules 7s et 19s ou sous forme de fragments obtenus par des procédés connus.

7

**4.** Procédé de production d'une préparation d'hyperimmunoglobulines polyvalente présentant une activité vis-à-vis des germes à Gram négatif et à Gram positif selon la revendication 1, caractérisé en ce que le plasma et/ou le sérum et/ou le sang entier de donneurs non vaccinés préalablement, est trié selon son titre en anticorps anti-antigène J5 d'E. coli ou anti-antigène lipide A, le plasma et/ou le sérum et/ou le sang entier ayant un titre dépassant la moyenne d'anticorps anti-antigène J5 d'E. coli ou anti-antigène lipide A est purifié et la préparation d'hyperimmunoglobulines polyvalente est obtenue à partir du pool ainsi constitué, selon les procédés traditionnels.

**5.** Procédé selon la revendication 4, caractérisé en ce qu'on utilise comme matériau de départ du plasma et/ou du sérum et/ou du sang entier, dont le titre correspond à une valeur d'au moins 1/160 en hémagglutination passive.